# EUROPÄISCHE PATENTSCHRIFT

(11) **EP 1 915 128 B1**
(45) Veröffentlichungstag und Bekanntmachung des Hinweises auf die Patenterteilung: **25.02.2009**
(21) Anmeldenummer: 06777917.3
(22) Anmeldetag: 24.07.2006
(51) Int. Cl.: A61K 9/00, A61K 9/06, A61K 47/32, A61K 47/24

(54) **STERILES TROPFBARES MEHRPHASIGES EMULGATORFREIES OPHTHALMISCHES PRÄPARAT**
STERILE, DROP-FORMING, MULTI-PHASE, EMULSIFIER-FREE OPHTHALMIC PRODUCT
PREPARATION OPHTALMOLOGIQUE STERILE, MULTIPHASE, EXEMPTE D'EMULSIFIANT, COULANT EN GOUTTES

(30) Priorität: 28.07.2005 DE 102005035986
(43) Veröffentlichungstag der Anmeldung: 30.04.2008
(73) Patentinhaber: Dr. GERHARD MANN chem.-pharm. Fabrik GmbH, 13581 Berlin (DE)
(72) Erfinder: CLAUS-HERZ, Gudrun, 14167 Berlin (DE); KESSLER, Christoph, 10623 Berlin (DE)
(74) Vertreter: Maiwald, Walter
(86) Internationale Anmeldenummer: PCT/EP2006/064557
(87) Internationale Veröffentlichungsnummer: WO 2007/012625

(56) Entgegenhaltungen:
- WO-A-00/01365
- WO-A-03/053452
- US-A1- 2004 082 660

## Beschreibung

Die Erfindung betrifft ein steriles tropfbares mehrphasiges emulgatorfreies ophthalmisches Präparat, insbesondere Gelpräparat. Das ophthalmische Präparat kann beispielsweise als künstliche Tränenflüssigkeit und zur Behandlung von "trockenem Auge" verwendet werden.

Es ist im Stand der Technik bekannt, bei gereizten oder entzündeten Augen, insbesondere bei "trockenem Auge", Keratokonjunktivitis sicca, wässrige Präparate zur Behandlung der Beschwerden einzusetzen. Diese Präparate liegen üblicherweise in Form von Flüssigkeiten, Gelen, Cremes oder Salben vor.

Nachteilig ist, dass Lösungen vielfach Inhaltsstoffe zugesetzt sind, die Allergien auslösen, zu Irritationen führen oder Sensibilisierungen oder Unverträglichkeiten gegenüber den Inhaltsstoffen erzeugen können. Auch ist von wässrigen Lösungen bekannt, dass diese häufig Jucken, Brennen oder andere unangenehme Nebenwirkungen beim Kontakt mit dem Auge erzeugen können.

Weiterhin haben insbesondere Präparate in Form von Gelen, Cremes oder Salben den Nachteil, dass das Auftragen von den Patienten, die bereits an einer schmerzhaften Reizung des Auges leiden, als zusätzlich unangenehm empfunden wird. Hinzu kommt, dass die Verteilung dieser Präparate im oder am Auge häufig durch Reiben unterstützt werden muss, ein Vorgang, der ebenfalls zu einem zusätzlichen Schmerzempfinden bei dem Patienten führt.

Auch zweiphasige Emulsionen sind im Stand der Technik bekannt. Deren Stabilität ist unter anderem jedoch von ihrer Viskosität abhängig. So werden Emulsion beispielsweise instabil, wenn eine in einer hydrophilen Phase feindispergierte hydrophobe Phase aggregiert. Dieser Vorgang schränkt die Stabilität bei einer Lagerung der Emulsionen ein und läuft umso langsamer ab, je viskoser die hydrophile Phase der Emulsion ist.

Um eine ausreichende Stabilität von Emulsionen zur Verfügung zu stellen, werden üblicherweise Emulgatoren eingesetzt. Emulgatoren können jedoch ebenfalls allergische oder auf Überempfindlichkeit des Patienten beruhende Reaktionen hervorrufen.

Die Zusammensetzung des Augenarzneimittels von WO 03/053452 (Gel, Salbe) verweist auf Phosphate wie Hyaluronate als Gelgerüstbildner. Der Wirkstoff kann in Vaselin oder Paraffin ohne den Zusatz eines Emulgators eingebettet werden. Die Zusammensetzung wird verwendet für die Herstellung eines Medikamentes zur Behandlung verschiedenster Ausgenkrankheiten.

WO 00/01365 offenbart ein ophthalmologisch einzusetzendes Gel, das Phosphate, Polyacrylsäure enthält und deren Viskosität zwischen 100 und 3000 mPa x s liegt. Die ophthalmologische Zusammensetzung von US 2004/082660 in Form einer Lösung, eines Gels, einer Augensalbe beinhaltet Phosphat als osmotisch wirksamen Hilfsstoff, Polyacrylat als Gelgerüstbildner und Glycerol als Lösungsmittel.

Ein emulgatorfreies Präparat ist beispielsweise in EP 0 817 610 A1 offenbart. Dieses Präparat hat jedoch den Nachteil, dass das Präparat eine sehr hohe Viskosität aufweist, so dass die Applikation des Präparats für den Patienten unangenehm ist und eine Unterstützung der

Verteilung des Präparats beispielsweise durch Reiben im Auge zu einem zusätzlichen Schmerzempfinden führt.

Es ist die Aufgabe der vorliegenden Erfindung eine Zusammensetzung zur Verfügung zu stellen, die wenigstens einen der vorgenannten Nachteile des Standes der Technik überwindet. Insbesondere ist es Aufgabe der vorliegenden Erfindung, eine Zusammensetzung zur Verfügung zu stellen, die zur Anwendung bei trockenem Augen geeignet ist.

Die Aufgabe wird gelöst durch ein steriles tropfbares mehrphasiges emulgatorfreies ophthalmisches Präparat, insbesondere Gelpräparat, enthaltend wenigstens eine flüssige wässrige Phase und wenigstens eine flüssige hydrophobe Phase, wobei das Präparat wenigstens ein Phosphatsalz enthält und eine Viskosität im Bereich von ≥ 200 mPa·s bis < 2000 mPa·s aufweist. Erfindungsgemäß bevorzugt geeignete Phosphatsalze sind wasserlöslich.

Vorteilhafte Ausgestaltungen des erfindungsgemäßen sterilen tropfbaren mehrphasigen emulgatorfreien ophthalmischen Präparats sind in den Unteransprüchen angegeben.

"Emulgatorfrei" bedeutet im Sinne dieser Erfindung, dass das Präparat ausschließlich Inhaltsstoffe aufweist, die keine Emulgatoren im Sinne der vorliegenden Erfindung sind. Insbesondere kann das Präparat gemäß der vorliegenden Erfindung Stoffe ausgewählt aus der Gruppe umfassend wasserlösliche Phosphatsalze, vorzugsweise Natriummonohydrogenphosphat-dodecahydrat, Na₂HPO₄ x 12 H₂O, Polyacrylsäure, polymere Acrylsäurederivate, vorzugsweise Carbomer, Triglyceride, vorzugsweise mittelkettige Triglyceride, eine Vitamin A-Komponente, vorzugsweise Vitamin A-palmitat, Vitamin E, Sorbitol, Natriumhydroxid, Konservierungsmittel wie Cetrimid, Benzododeciniumchlorid, Benzalkoniumchlorid oder Thiomersal und/oder Wasser aufweisen, wobei es sich bei diesen Stoffen im Sinne der vorliegenden Erfindung nicht um Emulgatoren handelt. Weiterhin kann das Präparat gemäß der vorliegenden Erfindung Stoffe ausgewählt aus der Gruppe umfassend Glycerol und/oder Alexidin aufweisen, wobei es sich bei diesen Stoffen im Sinne der vorliegenden Erfindung nicht um Emulgatoren handelt.

"Emulgatoren" im Sinne der vorliegenden Erfindung sind sind grenzflächenaktive Substanzen, die in der Lage sind, die Grenzflächenspannung zwischen Öl- und Wasserphase zu vermindern, indem sie sich bevorzugt an der Grenzfläche zwischen diesen beiden anlagern. Dies wird durch den amphipilen Molekülaufbau der Emulgatoren ermöglicht, die wenigstens eine polare (hydrophile) Gruppe und wenigstens eine unpolare (lipophile) Gruppe besitzen. Damit sind sie sowohl in der hydrophilen als auch in der lipophilen Phase löslich. Der in der entsprechenden Phase besser lösliche Teil ragt in diese hinein und senkt dadurch die Grenzflächenspannung zwischen beiden Phasen.

Eine bevorzugte Ausführungsform ist ein Präparat, insbesondere Gelpräparat, enthaltend wenigstens eine flüssige wässrige Phase und wenigstens eine flüssige hydrophobe Phase, wobei das Präparat wenigstens ein vorzugsweise wasserlösliches Phosphatsalz enthält und eine Viskosität im Bereich von ≥ 200 mPa·s bis < 2000 mPa·s aufweist, wobei das Präparat darüber hinaus keine weiteren Emulgatoren enthält oder in Mengen, dass die vorgenannten Stoffe keine emulgierende Wirkung aufweisen.

Eine weitere bevorzugte Ausführungsform ist ein Präparat, insbesondere Gelpräparat, enthaltend wenigstens eine flüssige wässrige Phase und wenigstens eine flüssige hydrophobe Phase, wobei das Präparat wenigstens ein vorzugsweise wasserlösliches Phosphatsalz enthält und eine Viskosität im Bereich von > 200 mPa·s bis < 2000 mPa·s aufweist, und wobei das Präparat Stoffe ausgewählt aus der Gruppe umfassend Polyacrylsäure, vorzugsweise ein Carbomer, mittelkettige Triglyceride, Natriummonohydrogenphosphat-dodecahydrat, Na₂HPO₄ x 12H₂O, Sorbitol, Natriumhydroxid und Wasser aufweist, wobei das Präparat darüber hinaus keine weiteren Emulgatoren enthält oder in Mengen, dass die vorgenannten Stoffe keine emulgierende Wirkung aufweisen.

Eine noch weiter bevorzugte Ausführungsform ist ein Präparat, insbesondere Gelpräparat, enthaltend wenigstens eine flüssige wässrige Phase und wenigstens eine flüssige hydrophobe Phase, wobei das Präparat wenigstens ein vorzugsweise wasserlösliches Phosphatsalz enthält und eine Viskosität im Bereich von ≥ 200 mPa·s bis < 2000 mPa·s aufweist, und wobei das Präparat Stoffe ausgewählt aus der Gruppe umfassend Polyacrylsäure, vorzugsweise ein Carbomer, mittelkettige Triglyceride, Natriummonohydrogenphosphat-dodecahydrat, Na₂HPO₄ x 12 H₂O, Sorbitol, Natriumhydroxid, Wasser und gegebenenfalls eine Vitamin A-Komponente, vorzugsweise Vitamin A-palmitat, Vitamin E, und/oder Konservierungsmittel, vorzugsweise Cetrimid, aufweist, wobei das Präparat darüber hinaus keine weiteren Emulgatoren enthält oder in Mengen, dass die vorgenannten Stoffe keine emulgierende Wirkung aufweisen.

Die Stoffe, die das Präparat gemäß der vorliegenden Erfindung enthalten kann, insbesondere wasserlösliche Phosphatsalze, Polyacrylsäure, polymere Acrylsäurederivate, vorzugsweise Carbomer, Triglyceride, eine Vitamin A-Komponente, Vitamin E, Sorbitol, Natriumhydroxid, Konservierungsmittel wie Cetrimid, Benzododeciniumchlorid, Benzalkoniumchlorid oder Thiomersal oder Wasser, werden in Mengen eingesetzt, dass die vorgenannten Stoffe keine emulgierende Wirkung aufweisen. Weiterhin kann das Präparat gemäß der vorliegenden Erfindung die Stoffe Glycerol und/oder Alexidin enthalten, die ebenfalls in Mengen eingesetzt werden, dass die vorgenannten Stoffe keine emulgierende Wirkung aufweisen.

Es hat sich überraschend gezeigt, dass das erfindungsgemäße Präparat, insbesondere Gelpräparat, in einem Viskositätsbereich < 2000 mPa·s stabil formuliert werden kann. Dies ist für den Fachmann insbesondere bei Emulsion solch niedriger Viskosität enthaltend eine hydrophile, beispielsweise wässrige, Phase und eine hydrophobe Phase überraschend, da diese üblicherweise rasch instabil werden und sich entmischen.

Ein besonderer Vorteil der Verbindungen ergibt sich hierbei dadurch, dass die Stabilität des Präparats ohne Zusatz von Emulgatoren erzielt werden kann. Dies ermöglicht eine Verbesserung der Verträglichkeit des erfindungsgemäßen Präparats.

Die Verwendung von Phosphatsalzen ermöglicht insbesondere, die erfindungsgemäß niedrigen Bereiche der Viskosität einzustellen. Ein besonderer Vorteil des erfindungsgemäßen Präparats wird dadurch verwirklicht, dass durch die Verwendung des Phosphatsalzes ein emulgatorfreies Präparat mit geringer Viskosität zur Verfügung gestellt werden kann.

Insbesondere ist von Vorteil, dass ein Präparat mit geringer Viskosität die Applizierbarkeit für den Patienten deutlich verbessert. Vorteilhafter Weise kann ein tropfbares Präparat zur Verfügung gestellt werden, das sich aufgrund der geringen Viskosität auf der Cornea des Auges verbessert verteilen kann, und das Auge weniger durch eine zusätzliche mechanische Unterstützung der Verteilung gereizt werden muss. Von großem Vorteil ist, dass das Präparat der erfindungsgemäß geringen Viskosität bei der Applikation ein nur geringes Fremdkörpergefühl im Auge erzeugt und für den Patienten eine angenehmere Verträglichkeit bereit stellt.

In bevorzugten Ausführungsformen des ophthalmischen Präparats weist das Präparat eine Viskosität im Bereich von ≥ 300 mPa·s bis ≤ 1800 mPa·s, vorzugsweise im Bereich von ≥ 400 mPa·s bis ≤ 1500 mPa·s, bevorzugt im Bereich von ≥ 500 mPa·s bis ≤ 1400 mPa·s, besonders bevorzugt im Bereich von ≥ 600 mPa·s bis ≤ 1350 mPa·s, ganz besonders bevorzugt im Bereich von ≥ 650 mPa·s bis ≤ 1300 mPa·s, auf.

Das sterile tropfbare ophthalmische Präparat umfasst vorzugsweise wenigstens ein wasserlösliches Phosphatsalz. Verwendbare wasserlösliche Phosphatsalze sind vorzugsweise ausgewählt aus der Gruppe umfassend Alkaliphosphatsalze, Erdalkaliphosphatsalze, Ammoniumphosphatsalze, deren Mono- oder Dihydrogenphosphate und/oder Mischungen davon. Bevorzugt verwendbar sind wasserlösliche Phosphatsalze ausgewählt aus der Gruppe umfassend Natriumdihydrogenphosphat, Natriummonohydrogenphosphat, Kaliumdihydrogenphosphat, Kaliummonohydrogenphosphat und/oder Mischungen davon. Ganz besonders bevorzugt verwendbar ist Natriummonohydrogenphosphat.

Bevorzugt verwendbar können die Hydratverbindungen wasserlöslicher Phosphatsalze sein, vorzugsweise Hydratverbindungen ausgewählt aus der Gruppe umfassend Hydratverbindungen der Alkaliphosphatsalze, Erdalkaliphosphatsalze, Ammoniumphosphatsalze, deren Mono- oder Dihydrogenphosphate und/oder Mischungen davon, besonders bevorzugt Hydratverbindungen ausgewählt aus der Gruppe umfassend Hydratverbindungen von Natriumdihydrogenphosphat, Natriummonohydrogenphosphat, Kaliumdihydrogenphosphat, Kaliummonohydrogenphosphat und/oder Mischungen davon. Bevorzugt verwendbar sind Na₂HPO₄ x n H₂O mit n= 2, 7 oder 12 oder NaH₂PO₄ x n H₂O mit n= 1 oder 2.

Ein besonders bevorzugt verwendbares Phosphatsalz ist Natriummonohydrogenphosphat-dodecahydrat, Na₂HPO₄ x 12 H₂O. Die Verwendung einer möglichen Bezeichnung wie Natriummonohydrogenphosphat-dodecahydrat schließt im Sinne dieser Erfindung entsprechende Bezeichnungen wie Dinatriumhydrogenphosphat-dodecahydrat oder Dinatriumphosphat-dodecahydrat für den entsprechenden Stoff mit ein.

Vorzugsweise liegt ein verwendbarer Anteil an wasserlöslichem Phosphatsalz im Bereich von ≥ 0,01 Gew.-% bis ≤ 5,0 Gew.-%, bevorzugt im Bereich von ≥ 0,05 Gew.-% bis ≤ 0,5 Gew.-%, besonders bevorzugt im Bereich von ≥ 0,08 Gew.-% bis ≤ 0,2 Gew.-%, bezogen auf das Gesamtgewicht des Präparats. In besonders bevorzugten Ausführungsformen enthält das Präparat etwa 0,1 Gew.-% wasserlösliches Phosphatsalz, bezogen auf das Gesamtgewicht des Präparats. Die Mengenangabe bezieht sich hierbei, wenn nicht anders angegeben, auf die entsprechende Hydratverbindung.

Als Untergrenze hat sich ein Mindestanteil an 0,01 Gew.-% Na₂HPO₄ x 12 H₂O bezogen auf das Gesamtgewicht des Präparats als günstig erwiesen. Als geeignet hat sich ein Anteil an Na₂HPO₄ x 12 H₂O im Bereich von ≥ 0,01 Gew.-% bis ≤ 5,0 Gew.-%, bezogen auf das Gesamtgewicht des Präparats herausgestellt. Bevorzugt ist ein Anteil an Na₂HPO₄ x 12 H₂O im Bereich von ≥ 0,05 Gew.-% bis ≤ 0,5 Gew.-%, besonders bevorzugt im Bereich von ≥ 0,08 Gew.-% bis ≤ 0,2 Gew.-%, bezogen auf das Gesamtgewicht des Präparats verwendbar. In besonders bevorzugten Ausführungsformen enthält das Präparat etwa 0,1 Gew.-% Na₂HPO₄ x 12 H₂O, bezogen auf das Gesamtgewicht des Präparats.

Ein weiterer überraschender Effekt des erfindungsgemäßen Präparats ist, dass das Präparat in bevorzugten Ausführungsformen bei der Anwendung im Auge der dreiphasigen Zusammensetzung des natürlichen Tränenfilms im Auge, Mucinschicht, wässrige Phase und Lipidschicht, nahe kommt und den natürlichen Tränenfilm substituieren kann. Insbesondere wird dies in vorteilhafter Weise unabhängig davon ermöglicht, welche Phase des natürlichen Tränenfilms in zu geringem Maße vorhanden ist oder fehlt.

Vorzugsweise enthält das mehrphasige Präparat die wässrige Phase als kontinuierliche Phase, und die flüssige hydrophobe Phase, vorzugsweise eine flüssige Ölphase, als darin dispergierte Tröpfchen, vorzugsweise in Form feinstverteilter Tröpfchen. Das Präparat ist vorzugsweise wenigstens zweiphasig ausgebildet, kann in bevorzugten Ausführungsformen auch dreiphasig ausgebildet sein. Das Präparat umfasst vorzugsweise weiterhin eine polymere gelbildende Komponente in der wässrigen Phase. Entsprechend enthält das erfindungsgemäße Präparat vorzugsweise eine zweiphasige, eine flüssige wässrige Phase und eine flüssige hydrophobe Phase umfassende, Trägerflüssigkeit oder Gelbasis.

Die wässrige Phase des Präparats wird bevorzugt dadurch ausgebildet, dass das Präparat einen bedeutenden Wasseranteil aufweist, vorzugsweise umfasst das Präparat einen Wasseranteil im Bereich von ≥ 60 Gew.-% bis ≤ 98 Gew.-%, bezogen auf das Gesamtgewicht des Präparats, bevorzugt im Bereich von ≥ 90 Gew.-% bis ≤ 96 Gew.-%, besonders bevorzugt enthält das Präparat etwa ≥ 94 Gew.-% bis ≤ 95 Gew.-% Wasser. Bevorzugt verwendbar ist Wasser für Injektionszwecke.

Die wässrige Phase des Präparats kann in bevorzugten Ausführungsformen die wässrige Phase des natürlichen Tränenfilms, die die Hornhaut befeuchtet, nachbilden und eine reinigende und schützende Funktion ausüben.

Vorteilhafter Weise ist das erfindungsgemäße ophthalmische Präparat besonders gut verträglich. Weiterhin von Vorteil ist, dass das erfindungsgemäße ophthalmische Präparat zu keiner oder einer nur sehr geringen Reizung oder Unannehmlichkeit bei der Anwendung am Auge führt. Dies bedeutet einen großen Vorteil, da eine Applikation am Auge vielfach zu einem sehr unangenehmen Gefühl des "Brennens" am Auge führt.

Vorzugsweise enthält das Präparat weiterhin wenigstens eine polymere gelbildende Komponente, insbesondere eine Polyacrylsäure und/oder ein polymeres Acrylsäurederivat. Bevorzugte Polyacrylsäuren weisen beispielsweise ein Molekulargewicht im Bereich von etwa 3 bis 5 Millionen Dalton auf. Besonders bevorzugt verwendbare Polyacrylsäuren oder polymere Acrylsäurederivate sind die unter dem INCI-Bezeichnung Carbomer bezeichneten quervernetzten Acrylsäurepolymere, vorzugsweise hydrophobisch modifizierte quervernetzte Acrylsäurepolymere. Bevorzugte Carbomere sind unter dem Handelsnamen Carbopol® beispielsweise bei der Firma Noveon Inc. erhältlich. Besonders bevorzugte Carbomere sind Acrylsäure-Homopolymere, beispielsweise Carbopol®-Homopolymere, besonders bevorzugt verwendbar ist Carbopol® 980 NF, weiterhin bevorzugt verwendbar ist Carbopol® 940 NF. Die Begriffe Polyacrylsäure und polymere Acrylsäure werden im Sinne dieser Anmeldung synonym verwendet.

Vorzugsweise enthält das ophthalmische Präparat die polymere gelbildende Komponente, vorzugsweise Polyacrylsäure und/oder ein polymeres Acrylsäurederivat, insbesondere bevorzugt ein Carbomer, im Bereich von ≥ 0,1 Gew.-% bis ≤ 3 Gew.-%, bezogen auf das Gesamtgewicht des Präparats, bevorzugt im Bereich von ≥ 0,1 Gew.-% bis ≤ 1 Gew.-%, besonders bevorzugt im Bereich von ≥ 0,15 Gew.-% bis ≤ 0,5 Gew.-%, ganz besonders bevorzugt etwa 0,2 Gew.-%.

In bevorzugten Ausführungsformen umfasst das Präparat weiterhin ein ophthalmisch und/oder ophthalmologisch akzeptables, organisches Öl. Das ophthalmisch akzeptable organisches Öl bildet in diesen Ausführungsformen die flüssige hydrophobe Phase des erfindungsgemäßen Präparats aus. Verwendbar als flüssige hydrophobe Öl-Komponente sind beispielsweise Fettsäurederivate wie Fettsäureester, Triglyceride und Phthalsäureester. Bevorzugt weist das Präparat als ophthalmisch akzeptables, organisches Öl mittelkettige Triglyceride, besonders bevorzugt Triglyceride ausgebildet aus, bezogen auf das Gesamtgewicht der Fettsäuren, wenigstens 80 Gew.-%, bevorzugt wenigstens 90 Gew.-%, besonders bevorzugt wenigstens 95 Gew.-%, C₈-C₁₂-Fettsäuren, bevorzugt C₈-C₁₀-Fettsäuren, auf.

Ganz besonders bevorzugt weist das Präparat gesättigte mittelkettige Triglyceride auf. Weiter bevorzugt verwendbar sind Triglyceride ausgebildet aus wenigstens 80 Gew.-%, bevorzugt wenigstens 90 Gew.-%, besonders bevorzugt wenigstens 95 Gew.-%, bezogen auf das Gesamtgewicht der Fettsäuren, gesättigten C₈- und C₁₀-Fettsäuren. Besonders bevorzugt sind Capryl-Caprinsäure-Triglyceride, Mischungen ausgebildet aus veresterten gesättigten C₈- und C₁₀-Fettsäuren.

Verwendbare Triglyceride sind erhältlich in Form von synthetischen, halbsynthetischen oder natürlichen Ölen, wie Olivenöl, Palmöl, Mandelöl, Kokosnussöl oder deren Mischungen. Bevorzugt verwendbare Triglyceride sind erhältlich aus Kokosnussöl. Solche mittelkettigen Triglyceride werden beispielsweise aus dem Öl des Endosperms von Cocos nucifera L., vorzugsweise des festen getrockenen Teils, oder Elasis guineensis Jacq. hergestellt. Weiterhin bevorzugt verwendbare Triglyceride, vorzugsweise mittelkettige Triglyceride, sind Neutralöle, beispielsweise erhältlich unter dem Handelsnamen Myritol® 318. Insbesondere sind auch mittelkettige Triglyceride der Art bevorzugt, wie sie im Europäischen Arzneibuch, European Pharmacopoeia 5.0, 01/2005:0868, S. 2623, Triglycerida saturata media, beschrieben werden. Bevorzugt enthält der Säurebestandteil ein Gemisch mit einem Anteil von wenigstens 90 Gew.-%, bevorzugt wenigstens 94 Gew.-%, besonders bevorzugt wenigstens 95 Gew.-%, bezogen auf das Gesamtgewicht der Fettsäuren, n-Octansäure und n-Decansäure.

Ein Vorteil der bevorzugt verwendbaren mittelkettigen Triglyceride auf Basis einer Mischung ausgebildet aus gesättigten C₈- und C₁₀-Fettsäuren, bevorzugt Capryl-Caprinsäure-Triglyceride, ist, dass die gesättigten Fettsäuren eine verbesserte Stabilität des Lipidfilms und damit eine überraschend verbesserte längere Verweildauer des Präparats im Auge zur Verfügung stellen können.

Die mittelkettigen Triglyceride können vorteilhafter Weise die Verdunstung der wässrigen Komponente verhindern und einem zu raschen Ablaufen des Tränenfilms oder des Präparats vorbeugen.

Vorzugsweise enthält das sterile tropfbare ophthalmische Präparat ein ophthalmisch und/oder ophthalmologisch akzeptables, organisches Öl, bevorzugt ausgewählt aus der Gruppe der Fettsäurederivate umfassend Fettsäureester, Triglyceride und/oder Phthalsäureester, besonders bevorzugt mittelkettige Triglyceride, im Bereich von ≥ 0,5 Gew.-% bis ≤ 10 Gew.-%, bezogen auf das Gesamtgewicht des Präparats, bevorzugt im Bereich von ≥ 0,6 Gew.-% bis ≤ 5 Gew.-%, besonders bevorzugt im Bereich von ≥ 0,8 Gew.-% bis ≤ 2 Gew.-%, ganz besonders bevorzugt etwa 1 Gew.-%. In besonders bevorzugten Ausführungsformen enthält das sterile tropfbare ophthalmische Präparat Capryl-Caprinsäure-Triglycerid oder Triglyceride ausgebildet aus wenigstens 80 Gew.-%, bevorzugt wenigstens 90 Gew.-%, besonders bevorzugt wenigstens 95 Gew.-%, bezogen auf das Gesamtgewicht der Fettsäuren, gesättigten C₈- und C₁₀-Fettsäuren im Bereich von ≥ 0,5 Gew.-% bis ≤ 10 Gew.-%, bezogen auf das Gesamtgewicht des Präparats, bevorzugt im Bereich von ≥ 0,6 Gew.-% bis ≤ 5 Gew.-%, besonders bevorzugt im Bereich von ≥ 0,8 Gew.-% bis ≤ 2 Gew.-%, ganz besonders bevorzugt etwa 1 Gew.-%.

Bevorzugte Ausführungsformen des erfindungsgemäßen Präparats, insbesondere auf Gelbasis, mit Gehalten an Gelbildner in der wässrigen Phase im Bereich von ≥ 0,1 Gew.-% bis ≤ 3 Gew.-%, bezogen auf das Gesamtgewicht des Präparats, insbesondere etwa 0,2 Gew.-%, enthalten mittelkettiges Triglycerid im Bereich von ≥ 0,5 Gew.-% bis ≤ 10 Gew.-%, insbesondere etwa 1 Gew.-% mittelkettiges Triglycerid.

Ein weiterer besonderer Vorteil des Präparats wird dadurch verwirklicht, dass das Präparat einen entsprechenden Gehalt an Triglyceriden enthalten kann, ohne dass gleichzeitig Emulgatoren eingesetzt werden müssen. Das hat den großen Vorteil, dass Anteile an restlicher noch vorliegender natürlicher Tränenflüssigkeit des Auges nicht durch einen Emulgator zerstört werden.

Vorzugsweise liegen die Triglyceride in dem erfindungsgemäßen Präparat in Form von feinstverteilten Tröpfchen vor. Ohne auf eine bestimmte Theorie festgelegt zu sein, wird angenommen, dass das Vorliegen der Triglyceride in dem erfindungsgemäßen Präparat in Form von feinstverteilten Tröpfchen eine überraschende Stabilität auch über lange Lagerzeiten ohne Emulgatorgehalt ermöglicht.

Ein weiterer Vorteil des Präparats wird dadurch verwirklicht, dass das Präparat insbesondere in Einzeldosis-Einheiten auch ohne Konservierungsmittel nicht nur unter den Bedingungen gemäßigten Klimas (21 °C, 45% r. F., relative Luftfeuchtigkeit) oder mediterran-subtropischen Klimas (25°C, 40 % r. F.), sondern sogar unter sehr heißen und feuchten klimatischen Bedingungen, beispielsweise bei 25°C, 60 % r. F., bei 30°C, 70 % r. F. oder bei 40°C, 75 % r. F., sehr stabil ist und wenigstens drei Monate, vorzugsweise sechs Monate, bevorzugter neun Monate, noch bevorzugter zwölf Monate, weiter bevorzugt 18 Monate, noch weiter bevorzugt 26 Monate, kaum oder nur geringfügige Veränderungen im Hinblick auf pH-Wert, Osmolalität, Viskosität und Aussehen zeigt.

Das erfindungsgemäße Präparat kann vorzugsweise keine Arzneistoffe oder pharmazeutisch aktiven Substanzen wie antivirale Mittel, steroidale und nicht-steroidale entzündungshemmende Verbindungen oder Corticosteroide, Antibiotika, Antimykotika, Narkotika, entzündungshemmender Agenzien oder antiallergische Agenzien enthalten. Pharmazeutisch aktive Substanzen im Sinne der Erfindung sind insbesondere nicht Vitamin A und Vitamin E.

In bevorzugten Ausführungsformen kann das erfindungsgemäße Präparat weiterhin wenigstens einen ophthalmischen Wirkstoff enthalten, beispielsweise eine Vitamin A-Komponente, bevorzugt Vitamin A-palmitat. Über die Verwendung von Vitaminen wie Vitamin A und/und Vitamin E hinaus, sind vorzugsweise keine pharmazeutisch aktiven Komponenten in dem erfindungsgemäßen Präparat vorgesehen.

Der besonders bevorzugte Gehalt an Vitamin A-palmitat liegt bei 1000 I.E. Vitamin A-palmitat pro Gramm Präparat, wobei vorteilhafter Weise zusätzlich 20 % Stabilitätszuschlag verwendet werden. Bevorzugt verwendbares Vitamin A-palmitat weist 1 Mio. I. E. pro Gramm Vitamin A-palmitat auf und enthält vorzugsweise als Stabilisatoren butyliertes Hydroxyanisol und/oder butyliertes Hydroxytoluol. Vorzugsweise liegt der Gehalt an einer Vitamin A-Komponente, insbesondere Vitamin A-palmitat, im Bereich von ≥ 0,05 Gew.-% bis ≤ 0,5 Gew.-%, bevorzugt im Bereich von ≥ 0,08 Gew.-% bis ≤ 0,2 Gew.-%, besonders bevorzugt bei etwa 0,1 Gew.-%, unter Berücksichtigung des Stabilitätszuschlags bei etwa 0,12 Gew.-%, bezogen auf das Gesamtgewicht des Präparats.

Besonders bevorzugt ist die Vitamin A-Komponente in Kombination mit einem Antioxidans wie Vitamin E verwendbar. Die Vitamin A-Komponente wird bevorzugt mit wenigstens einem Antioxidans, bevorzugt Vitamin E, besonders bevorzugt D,L-α-Tocopherol, stabilisiert. Zur Stabilisierung ist besonders bevorzugt ein geringer Gehalt Antioxidans, bevorzugt Vitamin E, besonders bevorzugt D,L-α-Tocopherol, verwendbar, beispielsweise im Bereich von ≥ 0,002 Gew.-% bis ≤ 0,01 Gew.-%, bevorzugt im Bereich von ≥ 0,006 Gew.-% bis ≤ 0,008 Gew.-%, bezogen auf das Gesamtgewicht des Präparats.

Ein weiterer Vorteil ist, dass ein suspendierter bzw. in der Ölphase gelöster Wirkstoff wie eine Vitamin A-Komponente, gleichmäßiger verteilt werden kann. Die Benetzbarkeit von Objekten, die auf die Cornea aufgelegt werden, wie Kontaktschalen oder Frontlinsen ophthalmischer Geräte, wird dadurch verbessert.

In bevorzugten Ausführungsformen umfasst das Präparat ein oder mehrere Mittel zur Einstellung der Isotonizität, vorzugsweise mehrwertige Alkohole wie Dextrose, Glycerin, Propylenglycol, Sorbitol oder Mannitol, bevorzugt enthält das Präparat Sorbitol.

Vorzugsweise weist das Präparat das oder die Mittel zur Einstellung der Isotonizität in einer Menge auf, die ausreicht, um die Zusammensetzung isotonisch mit natürlicher Tränenflüssigkeit bereit zu stellen. In bevorzugten Ausführungsformen umfasst das Präparat Isotonierungsmittel, vorzugsweise Sorbitol im Bereich von ≥ 0,5 Gew.-% bis ≤ 10 Gew.-%, bezogen auf das Gesamtgewicht des Präparats, bevorzugt im Bereich von ≥ 1 Gew.-% bis ≤ 5 Gew.-%, besonders bevorzugt etwa 4 Gew.-%. In noch bevorzugten Ausführungsformen umfasst das Präparat Isotonierungsmittel, vorzugsweise Sorbitol im Bereich von ≥ 3,5 Gew.-% bis ≤ 4 Gew.-%, bevorzugt im Bereich von ≥ 3,6 Gew.-% bis ≤ 3,7 Gew.-%, bezogen auf das Gesamtgewicht des Präparats. Insbesondere kann das Präparat Sorbitol im Bereich von ≥ 3,6 Gew.-% bis ≤ 3,7 Gew.-%, besonders bevorzugt etwa 3,666 Gew.-%, bezogen auf das Gesamtgewicht des Präparats, umfassen, wenn das Präparat ebenfalls Glycerol umfasst.

In bevorzugten Ausführungsformen weist das Präparat eine Osmolalität im Bereich von ≥ 100 mosmol/kg bis ≤ 500 mosmol/kg, vorzugsweise im Bereich von ≥ 200 mosmol/kg bis ≤ 300 mosmol/kg, besonders bevorzugt im Bereich von ≥ 220 mosmol/kg bis ≤ 260 mosmol/kg, auf.

Vorzugsweise wird der pH-Wert des ophthalmischen Präparats mit Säuren und/oder Basen, bevorzugt mit Borsäure und/oder Natronlauge eingestellt, wobei die Verwendung einer 2 %igen bis 3 %igen Lösung von Natriumhydroxid in Wasser besonders vorteilhaft ist.

Das sterile tropfbare ophthalmische Präparat enthält bevorzugt Natriumhydroxid, vorzugsweise im Bereich von ≥ 0,02 Gew.-% bis ≤ 1 Gew.-%, bezogen auf das Gesamtgewicht des Präparats, weiter bevorzugt im Bereich von ≥ 0,05 Gew.-% bis ≤ 0,1 Gew.-%, besonders bevorzugt im Bereich von ≥ 0,06 Gew.-% bis ≤ 0,07 Gew.-%. Die Angabe bezieht sich, wenn nicht anders angegeben, auf den Feststoff Natriumhydroxid. In ganz besonders bevorzugten Ausführungsformen enthält das unkonservierte Präparat etwa 0,0665 Gew.-% Natriumhydroxid, bezogen auf das Gesamtgewicht des Präparats, und ein Präparat, das Konservierungsmittel enthält, enthält bevorzugt etwa 0,06333 Gew.-% Natriumhydroxid, bezogen auf das Gesamtgewicht des Präparats.

In bevorzugten Ausführungsformen umfasst das ophthalmische Präparat Stoffe ausgewählt aus der Gruppe umfassend Polyacrylsäure, polymere Acrylsäurederivate, vorzugsweise Carbomer, Triglyceride, vorzugsweise mittelkettige Triglyceride, wasserlösliche Phosphatsalze vorzugsweise Na₂HPO₄ x 12 H₂O, Sorbitol, Natriumhydroxid und/oder Wasser. In besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 4,0 Gew.-% Sorbitol, 0,1 Gew.-% Na₂HPO₄ x 12 H₂O, 0,0665 Gew.-% Natriumhydroxid und/oder 1,0 Gew.-% mittelkettige Triglyceride sowie ad 100 Gew.-% Wasser.

In weiterhin bevorzugten Ausführungsformen umfasst das ophthalmische Präparat Stoffe ausgewählt aus der Gruppe umfassend Polyacrylsäure, polymere Acrylsäurederivate, insbesondere Carbomere, Triglyceride, insbesondere mittelkettige Triglyceride, wasserlösliche Phosphatsalze, insbesondere Na₂HPO₄ x 12 H₂O, eine Vitamin A-Komponente, vorzugsweise Vitamin A-palmitat, Vitamin E, vorzugsweise D,L-α-Tocopherol, Sorbitol, Natriumhydroxid und/oder Wasser. In weiterhin besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 4,0 Gew.-% Sorbitol, 0,1 Gew.-% Na₂HPO₄ x 12 H₂O, 0,0665 Gew.-% Natriumhydroxid, 0,12 Gew.-% Vitamin A-palmitat, 0,006 Gew.-% Vitamin E, vorzugsweise D,L-α-Tocopherol und/oder 1,0 Gew.-% mittelkettige Triglyceride sowie ad 100 Gew.-% Wasser.

In vorteilhaften Ausführungsformen weist das Präparat einen pH-Wert im Bereich von ≥ 6 bis ≤ 8, bevorzugt im Bereich von ≥ 6,5 bis ≤ 7,5, auf. In besonders vorteilhaften Ausführungsformen weist das Präparat einen pH-Wert im Bereich von ≥ 6,8 bis ≤ 7,2 auf.

Das erfindungsgemäße Präparat kann weitere Komponenten enthalten, beispielsweise Konservierungsmittel, vorzugsweise ausgewählt aus der Gruppe umfassend Cetrimid, Benzododeciniumchlorid, Benzalkoniumchlorid und/oder Thiomersal. Bevorzugt sind die Konservierungsmittel ausgewählt aus der Gruppe umfassend Cetrimid und/oder Alexidin. Bevorzugt enthält das Präparat Konservierungsmittel, vorzugsweise ausgewählt aus der Gruppe umfassend Cetrimid, Benzododeciniumchlorid, Benzalkoniumchlorid und/oder Thiomersal, im Bereich von ≥ 0,001 Gew.-% bis ≤ 0,05 Gew.-%, bezogen auf das Gesamtgewicht des Präparats, besonders bevorzugt im Bereich von ≥ 0,005 Gew.-% bis ≤ 0,01 Gew.-%.

"Cetrimid" ist die übliche Bezeichnung für N-Cetyl-N,N,N-trimethyl-ammoniumbromid, "Benzododeciniumchlorid" für N-Benzyl-N-dodecyl-N,N-dimethyl-ammoniumchlorid, "Benzalkoniumchlorid" für Benzyllauryldimethylammoniumchlorid, "Thiomersal" für das Natriumsalz von 2-(Ethylmercurithio)-benzoesäure.

In bevorzugten Ausführungsformen umfasst das Präparat 0,01 Gew.-%, bezogen auf das Gesamtgewicht des Präparats, Cetrimid und/oder Benzododeciniumchlorid. In weiterhin bevorzugten Ausführungsformen umfasst das Präparat 0,01 Gew.-%, bezogen auf das Gesamtgewicht des Präparats, Benzalkoniumchlorid. In besonders bevorzugten Ausführungsformen ist das Präparat frei von Konservierungsmitteln.

Das erfindungsgemäße Präparat kann als weiteren Stoff Alexidin enthalten, bevorzugt Alexidinhydrochlorid, vorzugsweise kann das erfindungsgemäße Präparat etwa 3 ppm Alexidin · 2HCl, bezogen auf das Gesamtgewicht des Präparats, enthalten. Bei Alexidin handelt es sich im Sinne der vorliegenden Erfindung nicht um einen Emulgator, oder Alexidin ist in Mengen enthalten, dass der Stoff keine emulgierende Wirkung aufweist.

Als weiteren Stoff kann das erfindungsgemäße Präparat Glycerol, auch Glycerin genannt, enthalten, beispielsweise etwa 0,2 Gew.-%, bezogen auf das Gesamtgewicht des Präparats. Bei Glycerin handelt es sich im Sinne der vorliegenden Erfindung nicht um einen Emulgator, oder Glycerin ist in Mengen enthalten, dass der Stoff keine emulgierende Wirkung aufweist.

Vorzugsweise kann das Präparat Glycerol im Bereich von ≥ 0,01 Gew.-% bis ≤ 1,0 Gew.-%, bezogen auf das Gesamtgewicht des Präparats, bevorzugt im Bereich von ≥ 0,1 Gew.-% bis ≤ 0,5 Gew.-%, besonders bevorzugt im Bereich von ≥ 0,2 Gew.-% bis ≤ 0,3 Gew.-%, aufweisen. Das Präparat kann Glycerol in Form von 100 %igem Glycerol oder 85 %igem Glycerol, wobei es sich vorzugsweise um eine Mischung mit Wasser handelt, aufweisen. In bevorzugten Ausführungsformen kann das Präparat 0.2 Gew.-% 100 %iges Glycerol oder 0,235 Gew.-% 85 %iges Glycerol umfassen.

In bevorzugten Ausführungsformen umfasst das ophthalmische Präparat Stoffe ausgewählt aus der Gruppe umfassend Polyacrylsäure, polymere Acrylsäurederivate, vorzugsweise Carbomere, Triglyceride, vorzugsweise mittelkettige Triglyceride, wasserlösliche Phosphatsalze, vorzugsweise Na₂HPO₄ x 12 H₂O, eine Vitamin A-Komponente, vorzugsweise Vitamin A-palmitat, Vitamin E, vorzugsweise D,L-α-Tocopherol, Sorbitol, Natriumhydroxid, Konservierungsmittel, vorzugsweise Cetrimid, Benzododeciniumchlorid, Benzalkoniumchlorid oder Alexidin, Glycerol und/oder Wasser.

In noch bevorzugten Ausführungsformen umfasst das ophthalmische Präparat Stoffe ausgewählt aus der Gruppe umfassend Polyacrylsäure, polymere Acrylsäurederivate, insbesondere Carbomere, Triglyceride, insbesondere mittelkettige Triglyceride, wasserlösliche Phosphatsalze, insbesondere Na₂HPO₄ x 12 H₂O, Sorbitol, Natriumhydroxid, Glycerol und/oder Wasser. In weiterhin noch besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 3,666 Gew.-% Sorbitol, 0,1 Gew.-% Na₂HPO₄ x 12 H₂O, 0,063 Gew.-% Natriumhydroxid, 0,235 Gew.-% 85 %iges Glycerol oder 0,2 Gew.-% 100 %iges Glycerol und/oder 1,0 Gew.-% mittelkettige Triglyceride sowie ad 100 Gew.-% Wasser.

In noch weiter bevorzugten Ausführungsformen umfasst das ophthalmische Präparat Stoffe ausgewählt aus der Gruppe umfassend Polyacrylsäure, polymere Acrylsäurederivate, insbesondere Carbomere, Triglyceride, insbesondere mittelkettige Triglyceride, wasserlösliche Phosphatsalze, insbesondere Na₂HPO₄ x 12 H₂O, eine Vitamin A-Komponente, vorzugsweise Vitamin A-palmitat, Vitamin E, vorzugsweise D,L-α-Tocopherol, Sorbitol, Natriumhydroxid, Glycerol und/oder Wasser. In weiterhin noch besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 3,666 Gew.-% Sorbitol, 0,1 Gew.-% Na₂HPO₄ x 12 H₂O, 0,063 Gew.-% Natriumhydroxid, 0,12 Gew.-% Vitamin A-palmitat, 0,006 Gew.-% Vitamin E, vorzugsweise D,L-α-Tocopherol 0,235 Gew.-% 85 %iges Glycerol oder 0,2 Gew.-% 100 %iges Glycerol und/oder 1,0 Gew.-% mittelkettige Triglyceride sowie ad 100 Gew.-% Wasser.

In weiterhin bevorzugten Ausführungsformen umfasst das ophthalmische Präparat Stoffe ausgewählt aus der Gruppe umfassend Polyacrylsäure, polymere Acrylsäurederivate, vorzugsweise Carbomer, Triglyceride, vorzugsweise mittelkettige Triglyceride, wasserlösliche Phosphatsalze, vorzugsweise Na₂HPO₄ x 12 H₂O, ein Konservierungsmittel, vorzugsweise Cetrimid, Sorbitol, Natriumhydroxid und/oder Wasser. In weiterhin besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 4,0 Gew.-% Sorbitol, 0,1 Gew.-% Na₂HPO₄ x 12 H₂O, 0,06333 Gew.-% Natriumhydroxid,

0,1 Gew.-% Cetrimid und/oder 1,0 Gew.-% mittelkettige Triglyceride sowie ad 100 Gew.-% Wasser. In auch besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 4,0 Gew.-% Sorbitol, 0,1 Gew.-% Na₂HPO₄ x 12 H₂O, 0,06333 Gew.-% Natriumhydroxid, 0,01 Gew.-% Cetrimid und/oder 1,0 Gew.-% mittelkettige Triglyceride sowie ad 100 Gew.-% Wasser.

In noch bevorzugten Ausführungsformen umfasst das ophthalmische Präparat Stoffe ausgewählt aus der Gruppe umfassend Polyacrylsäure, polymere Acrylsäurederivate, vorzugsweise Carbomere, Triglyceride, vorzugsweise mittelkettige Triglyceride, wasserlösliche Phosphatsalze, vorzugsweise Na₂HPO₄ x 12 H₂O, eine Vitamin A-Komponente, vorzugsweise Vitamin A-palmitat, Vitamin E, vorzugsweise D,L-α-Tocopherol, Sorbitol, Natriumhydroxid, Konservierungsmittel, vorzugsweise Cetrimid oder Benzododeciniumchlorid und/oder Wasser. In weiterhin besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 4,0 Gew.-% Sorbitol, 0,1 Gew.-% Na₂HPO₄ x 12 H₂O, 0,06333 Gew.-% Natriumhydroxid, 0,1 Gew.-% Cetrimid, 0,12 Gew.-% Vitamin A-palmitat, 0,006 Gew.-% Vitamin E, vorzugsweise D,L-α-Tocopherol und/oder 1,0 Gew.-% mittelkettige Triglyceride sowie ad 100 Gew.-% Wasser. In auch weiterhin besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 4,0 Gew.-% Sorbitol, 0,1 Gew.-% Na₂HPO₄ x 12 H₂O, 0,06333 Gew.-% Natriumhydroxid, 0,01 Gew.-% Cetrimid, 0,12 Gew.-% Vitamin A-palmitat, 0,006 Gew.-% Vitamin E, vorzugsweise D,L-α-Tocopherol und/oder 1,0 Gew.-% mittelkettige Triglyceride sowie ad 100 Gew.-% Wasser.

In noch weiter bevorzugten Ausführungsformen umfasst das ophthalmische Präparat Stoffe ausgewählt aus der Gruppe umfassend Polyacrylsäure, polymere Acrylsäurederivate, vorzugsweise Carbomere, Triglyceride, vorzugsweise mittelkettige Triglyceride, wasserlösliche Phosphatsalze, vorzugsweise Na₂HPO₄ x 12 H₂O, Glycerol, Sorbitol, Natriumhydroxid, Konservierungsmittel, vorzugsweise Cetrimid oder Benzalkoniumchlorid und/oder Wasser. In noch weiterhin besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 3,666 Gew.-% Sorbitol, 0,1 Gew.-% Na₂HPO₄ x 12 H₂O, 0,063 Gew.-% Natriumhydroxid, 0,01 Gew.-% Cetrimid oder 0,01 Gew.-% Benzalkoniumchlorid, 0,2 Gew.-% 100 %iges Glycerol oder 0,235 Gew.-% 85 %iges Glycerol und/oder 1,0 Gew.-% mittelkettige Triglyceride sowie ad 100 Gew.-% Wasser.

In auch bevorzugten Ausführungsformen umfasst das ophthalmische Präparat Stoffe ausgewählt aus der Gruppe umfassend Polyacrylsäure, polymere Acrylsäurederivate, vorzugsweise Carbomere, Triglyceride, vorzugsweise mittelkettige Triglyceride, wasserlösliche Phosphatsalze, vorzugsweise Na₂HPO₄ x 12 H₂O, Glycerol, Sorbitol, Natriumhydroxid, Konservierungsmittel, vorzugsweise Alexidin und/oder Wasser. In auch besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 3,666 Gew.-% Sorbitol, 0,1 Gew.-% Na₂HPO₄ x 12 H₂O, 0,063 Gew.-% Natriumhydroxid, 0,0003 Gew.-% Alexidin HCl, 0,2 Gew.-% 100 %iges Glycerol oder 0,235 Gew.-% 85 %iges Glycerol und/oder 1,0 Gew.-% mittelkettige Triglyceride sowie ad 100 Gew.-% Wasser.

In besonders bevorzugten Ausführungsformen umfasst das ophthalmische Präparat, bezogen auf das Gesamtgewicht des Präparats, die nachstehenden Stoffe:
a. 0,2 Gew.-% polymere Acrylsäure;
b. 4,0 Gew.-% Sorbitol;
c. 0,1 Gew.-% Na₂HPO₄ x 12 H₂O;
d. 0,0665 Gew.-% Natriumhydroxid;
e. 1,0 Gew.-% mittelkettige Triglyceride;
f. ad 100 Gew.-% Wasser.

Wenn nicht anders angegeben, sind die Gewichtsgehalte der jeweiligen im Präparat enthaltenen Stoffe so gewählt, dass das Gesamtgewicht der jeweiligen Stoffe 100 Gew.-%, bezogen auf das Gesamtgewicht des Präparats, nicht übersteigt.

Unter dem Begriff "Stoff" sind im Sinne dieser Erfindung die Stoffe zu verstehen, die das Präparat aufweist, insbesondere ausgewählt aus der Gruppe umfassend polymere Acrylsäure, polymeres Acrylsäurederivat, Carbomer, Sorbitol, wasserlösliches Phosphatsalz, vorzugsweise Na₂HPO₄ x 12 H₂O, Natriumhydroxid, mittelkettige Triglyceride, Konservierungsmittel, vorzugsweise Cetrimid, Vitamin A-Komponente, vorzugsweise Vitamin A-palmitat, Vitamin E, vorzugsweise D,L-α-Tocopherol, Glycerol und/oder Wasser.

In weiterhin bevorzugten Ausführungsformen umfasst das ophthalmische Präparat, bezogen auf das Gesamtgewicht des Präparats, die nachstehenden Stoffe:
a. 0,2 Gew.-% polymere Acrylsäure;
b. 4,0 Gew.-% Sorbitol;
c. 0,1 Gew.-% Na₂HPO₄ x 12 H₂O;
d. 0,06333 Gew.-% Natriumhydroxid;
e. 1,0 Gew.-% mittelkettige Triglyceride;
f. 0,1 Gew.-% Cetrimid;
g. ad 100 Gew.-% Wasser.

In noch weiter bevorzugten Ausführungsformen umfasst das ophthalmische Präparat, bezogen auf das Gesamtgewicht des Präparats, die nachstehenden Stoffe:
a. 0,2 Gew.-% polymere Acrylsäure;
b. 4,0 Gew.-% Sorbitol;
c. 0,1 Gew.-% Na₂HPO₄ x 12 H₂O;
d. 0,06333 Gew.-% Natriumhydroxid;
e. 1,0 Gew.-% mittelkettige Triglyceride;
f. 0,1 Gew.-% Cetrimid;
g. 0,12 Gew.-% Vitamin A-palmitat;
h. 0,006 Gew.-% D,L-α-Tocopherol;
i. ad 100 Gew.-% Wasser.

In ebenfalls bevorzugten Ausführungsformen umfasst das ophthalmische Präparat, bezogen auf das Gesamtgewicht des Präparats, die nachstehenden Stoffe:
a. 0,2 Gew.-% polymere Acrylsäure;
b. 4,0 Gew.-% Sorbitol;
c. 0,1 Gew.-% Na₂HPO₄ x 12 H₂O;
d. 0,06333 Gew.-% Natriumhydroxid;
e. 1,0 Gew.-% mittelkettige Triglyceride;
f. 0,01 Gew.-% Cetrimid;
g. ad 100 Gew.-% Wasser.

In ebenfalls weiter bevorzugten Ausführungsformen umfasst das ophthalmische Präparat, bezogen auf das Gesamtgewicht des Präparats, die nachstehenden Stoffe:
a. 0,2 Gew.-% polymere Acrylsäure;
b. 4,0 Gew.-% Sorbitol;
c. 0,1 Gew.-% Na₂HPO₄ x 12 H₂O;
d. 0,06333 Gew.-% Natriumhydroxid;
e. 1,0 Gew.-% mittelkettige Triglyceride;
f. 0,01 Gew.-% Cetrimid;
g. 0,12 Gew.-% Vitamin A-palmitat;
h. 0,006 Gew.-% D,L-α-Tocopherol;
i. ad 100 Gew.-% Wasser.

In auch bevorzugten Ausführungsformen umfasst das ophthalmische Präparat, bezogen auf das Gesamtgewicht des Präparats, die nachstehenden Stoffe:
a. 0,2 Gew.-% polymere Acrylsäure;
b. 3,666 Gew.-% Sorbitol;
c. 0,1 Gew.-% Na₂HPO₄ x 12 H₂O;
d. 0,063 Gew.-% Natriumhydroxid;
e. 1,0 Gew.-% mittelkettige Triglyceride;
f. 0,01 Gew.-% Benzalkoniumchlorid;
g. 0,2 Gew.-% Glycerol (100 %), oder
   0,235 Gew.-% Glycerol (85 %);
h. ad 100 Gew.-% Wasser.

In auch noch bevorzugten Ausführungsformen umfasst das ophthalmische Präparat, bezogen auf das Gesamtgewicht des Präparats, die nachstehenden Stoffe:
a. 0,2 Gew.-% polymere Acrylsäure;
b. 3,666 Gew.-% Sorbitol;
c. 0,1 Gew.-% Na₂HPO₄ x 12 H₂O;
d. 0,063 Gew.-% Natriumhydroxid;
e. 1,0 Gew.-% mittelkettige Triglyceride;
f. 0,0003 Gew.-% Alexidine HCl;
g. 0,2 Gew.-% Glycerol (100 %), oder
   0,235 Gew.-% Glycerol (85 %);
h. ad 100 Gew.-% Wasser.

In weiter auch bevorzugten Ausführungsformen umfasst das ophthalmische Präparat, bezogen auf das Gesamtgewicht des Präparats, die nachstehenden Stoffe:
a. 0,2 Gew.-% polymere Acrylsäure;
b. 3,666 Gew.-% Sorbitol;
c. 0,1 Gew.-% Na₂HPO₄ x 12 H₂O;
d. 0,063 Gew.-% Natriumhydroxid;
e. 1,0 Gew.-% mittelkettige Triglyceride;
f. 0,2 Gew.-% Glycerol (100 %), oder
   0,235 Gew.-% Glycerol (85 %);
g. ad 100 Gew.-% Wasser.

Das erfindungsgemäße Präparat lässt sich vorteilhafter Weise unproblematisch aseptisch herstellen und ist hervorragend verträglich. Die Herstellung des erfindungsgemäßen sterilen Präparats, insbesondere Gelpräparats, erfolgt vorzugsweise in einem mehrstufigen Verfahren. Insbesondere wird der wässrigen Phase des erfindungsgemäßen Präparats wenigstens ein wasserlösliches Phosphatsalz zusetzt.

Es versteht sich von selbst, dass das ophthalmische Präparat zur Verwendung vorzugsweise steril vorliegt, so dass sterile Stoffe unter sterilen Bedingungen verwendet werden, oder das Präparat nach der Einarbeitung der Stoffe sterilisiert wird.

Bei einem bevorzugten Verfahren zur Herstellung eines erfindungsgemäßen Präparats wird eine Suspension der polymeren Acrylsäure oder des polymeren Acrylsäurederivates, insbesondere eines Carbomers, hergestellt, bevorzugt unter aseptischen Bedingungen. Zu dieser Suspension wird anschließend eine wässrige sterilfiltrierte Lösung des Isotonisierungsmittels, vorzugsweise Sorbitol, enthaltend ebenfalls Natriummonohydrogenphosphat-dodecahydrat oder andere Phosphatsalze, und gegebenenfalls ein Konservierungsmittel gegeben, wobei unter Verwendung von bevorzugt sterilfiltiertem Stickstoff oder Druckluft als Druckgas gearbeitet werden kann. Nach sorgfältigem Durchmischen werden dann durch Zugabe einer geeigneten Base, vorzugsweise einer sterilen 2 %igen bis 3 %igen Natriumhydroxidlösung, die Carboxylgruppen der Polyacrylsäurekomponente neutralisiert, die Gelbildung der Polyacrylsäurekomponente eingeleitet und es wird vorzugsweise bis zur Homogenität des Gels weitergerührt.

In das hergestellte sterile Hydrogel wird dann die hydrophobe flüssige Phase, vorzugsweise die mittelkettige Triglyceridkomponente, unter aseptischen Bedingungen eingearbeitet. Vorzugsweise wird das mittelkettige Triglycerid homogen in der kontinuierlichen wässrigen Phase, insbesondere dem sterilen Hydrogel, dispergiert.

Bevorzugt wird bis zur vollständigen Homogenisierung gerührt. Die Größe der so erzeugten Tröpfchen der hydrophobe flüssige Phase, oder Öltröpfchen, in der Dispersion liegt vorzugsweise bei maximal etwa 100 µm. Das sterile Präparat kann anschließend in üblicher Weise konfektioniert werden.

Bei dem Verfahren zur Herstellung eines erfindungsgemäßen Präparats kann ebenfalls ein Wirkstoff, wie Vitamin A-palmitat, dem so hergestellten sterilen Gel zugesetzt werden. Bevorzugt wird der Wirkstoff unter aseptischen Bedingungen zugesetzt und homogen eingemischt. Vorzugsweise wird die Vitamin A-Komponente und die vorzugsweise vorhandene sehr viel geringere Menge des Antioxidans in der hydrophoben Phase gelöst und dann steril filtriert. Die.sterile hydrophobe wirkstoffhaltige Phase wird dann unter Rühren in das Gel eingearbeitet.

Von besonderem Vorteil ist, dass das erfindungsgemäße ophthalmische Präparat zur Herstellung eines kosmetischen und/oder pharmazeutischen Mittels zur Behandlung von Krankheiten oder Zuständen des Auges oder der das Auge umgebenden oder damit zusammenhängenden Organe oder Gewebe, insbesondere von trockenem Auge verwendbar ist.

Durch die vorteilhaften Stoffe des Präparats kann dieses als Mittel zur Behandlung von Krankheiten oder Zuständen des Auges oder der das Auge umgebenden oder damit zusammenhängenden Organe oder Gewebe, insbesondere in Form eines Gelpräparats, eingesetzt werden. Insbesondere ist das Präparat zur Linderung der Beschwerden bei trockenem Auge und/oder für die symptomatische Behandlung des trockenen Auges geeignet.

Insbesondere das konservierungsmittelfreie erfindungsgemäße ophthalmische Präparat ist in vorteilhafter Weise in der Langzeitanwendung bei trockenem Auge verwendbar. Durch die vorteilhaften Stoffe des ophthalmischen Präparats kann insbesondere das konservierungsmittelfreie ophthalmische Präparat unbedenklich zur andauernden Anwendung beispielsweise als Tränenersatzmittel Verwendung finden.

Es wurde überraschend gefunden, dass schon innerhalb weniger Minuten nach der Applikation des Präparats eine deutliche Besserung der Beschwerden wie Trockenheitsgefühl am Auge, aber auch Brennen, Jucken, Rötungen oder Schwellungen, bewirkt werden kann. Von besonderem Vorteil ist, dass diese Beschwerden nachhaltig durch das erfindungsgemäße Präparat gelindert werden können.

Für die Applikation wird das erfindungsgemäße Präparat vorzugsweise in geeignete Behältnisse abgefüllt, die vorzugsweise so gestaltet sind, dass der Inhalt auf das Auge aufgebracht werden kann, beispielsweise in Tropflaschen, insbesondere in unter der Bezeichnung Ophtiole® bekannte Behältnisse, vorzugsweise basierend auf Polyethylen, bevorzugt HDPE (Polyethylen hoher Dichte, high density Polyethylen) oder LDPE (Polyethylen niederer Dichte, low density Polyethylen).

In bevorzugten Ausführungsformen des ophthalmischen Präparats ist dieses in einer Eindosis-Einheit, vorzugsweise einem Einmaldosis-Behältnis beispielsweise bekannt unter der Bezeichnung Eindosis-Ophtiole®, vorzugsweise basierend auf LDPE, enthalten. In besonders bevorzugten Ausführungsformen des ophthalmischen Präparats liegt dieses in Form eines Gelpräparats in einem Einmaldosis-Behältnis frei von Konservierungsmitteln vor.

In weiterhin bevorzugten Ausführungsformen des sterilen tropfbaren mehrphasigen emulgatorfreien ophthalmischen Präparats ist dieses in einer Mehrdosen-Einheit, beispielsweise einem Mehrdosen-Behältnis enthalten. In auch besonders bevorzugten Ausführungsformen des ophthalmischen Präparats liegt dieses vorzugsweise in Form eines Gelpräparats in einem Mehrdosen-Behältnis frei von Konservierungsmitteln vor.

Eine Mehrdosen-Einheit enthält eine größere Menge des sterilen tropfbaren mehrphasigen emulgatorfreien ophthalmischen Präparats, beispielsweise mehrere Dosen. Somit ist vorteilhafter Weise möglich, dass beispielsweise eine Mehrfachdosis in einem Behältnis enthalten sein kann.

Die Viskosität wurde bestimmt nach dem Platte-Kegel-Verfahren mit einem Rheometer des Typs DV-III+ der Firma Brookfield, unter Verwendung einer CP51-Spindel, bei 5 U/min, bei 20 °C. Angaben der Viskosität beziehen sich, wenn nicht anders angegeben, auf die Viskosität des Präparats vor der Applikation am Auge.

Das folgende Ausführungsbeispiel dient der Erläuterung der Erfindung und stellt keinerlei Einschränkung dar.

### Beispiel 1

Augengel umfassend, bezogen auf 100 kg:
- 0,2 kg polymere Acrylsäure*¹;
- 4,0 kg Sorbitol;
- 0,1 kg Na₂HPO₄ x 12 H₂O;
- 0,0665 kg Natriumhydroxid;
- 1 kg mittelkettige Triglyceride *²;
- ad 100 kg Wasser.

* ¹ Beispielsweise erhältlich unter dem Handelsnamen Carbopol® 980 NF bei der Firma Noveon Inc..
* ² Beispielsweise erhältlich unter dem Handelsnamen Myritol 318®.

Das Präparat wies eine Viskosität von 781 mPa·s, bestimmt nach dem Platte-Kegel-Verfahren mit einem Rheometer des Typs DV-III+ der Firma Brookfield, unter Verwendung einer CP51-Spindel, bei 5 U/min, bei 20 °C, einen pH-Wert von 7,0 und eine Osmolalität von 261 mosmol/kg auf.

## Patentansprüche

1. Steriles tropfbares mehrphasiges emulgatorfreies ophthalmisches Präparat, insbesondere Gelpräparat, enthaltend wenigstens eine flüssige wässrige Phase und wenigstens eine flüssige hydrophobe Phase,
**dadurch gekennzeichnet, dass**
das Präparat wenigstens ein Phosphatsalz enthält und eine Viskosität im Bereich von ≥ 200 mPa·s bis < 2000 mPa·s aufweist, bestimmt nach dem Platte-Kegel-Verfahren bei 20°C.

2. Steriles tropfbares mehrphasiges emulgatorfreies ophthalmisches Präparat nach Anspruch 1, **dadurch gekennzeichnet, dass**
die Viskosität im Bereich von ≥ 300 mPa·s bis ≤ 1800 mPa·s, vorzugsweise im Bereich von ≥ 400 mPa·s bis ≤ 1500 mPa·s, bevorzugt im Bereich von ≥ 500 mPa·s bis ≤ 1400 mPa·s, besonders bevorzugt im Bereich von ≥ 600 mPa·s bis ≤ 1350 mPa·s, ganz besonders bevorzugt im Bereich von ≥ 650 mPa·s bis ≤ 1300 mPa·s, liegt.

3. Steriles tropfbares mehrphasiges emulgatorfreies ophthalmisches Präparat nach Anspruch 1 oder 2, **dadurch gekennzeichnet, dass**
ein wasserlösliches Phosphatsalz ausgewählt ist aus der Gruppe umfassend Alkaliphosphatsalze, Erdalkaliphosphatsalze, Ammoniumphosphatsalze, deren Mono- oder Dihydrogenphosphate und/oder Mischungen davon, bevorzugt ausgewählt aus der Gruppe umfassend Natriumdihydrogenphosphat, Natriummonohydrogenphosphat, Kaliumdihydrogenphosphat, Kaliummonohydrogenphosphat und/oder Mischungen davon.

4. Steriles tropfbares mehrphasiges emulgatorfreies ophthalmisches Präparat nach einem der vorherigen Ansprüche, **dadurch gekennzeichnet, dass**
das Präparat Na₂HPO₄ x 12 H₂O im Bereich von ≥ 0,01 Gew.-% bis ≤ 5,0 Gew.%, bezogen auf das Gesamtgewicht des Präparats, bevorzugt im Bereich von ≥ 0,05 Gew.% bis ≤ 0,5 Gew.-%, vorzugsweise im Bereich von ≥ 0,08 Gew.-% bis ≤ 0,2 Gew.-%, besonders bevorzugt etwa 0,1 Gew.-% Na₂HPO₄ x 12 H₂O, enthält.

5. Steriles tropfbares mehrphasiges emulgatorfreies ophthalmisches Präparat nach einem der vorherigen Ansprüche, **dadurch gekennzeichnet, dass**
das Präparat die wässrige Phase als kontinuierliche Phase und die hydrophobe Phase als darin dispergierte Tröpfchen enthält.

6. Steriles tropfbares mehrphasiges emulgatorfreies ophthalmisches Präparat nach einem der vorherigen Ansprüche, **dadurch gekennzeichnet, dass**
das Präparat wenigstens eine polymere gelbildende Komponente, vorzugsweise eine Polyacrylsäure und/oder ein polymeres Acrylsäurederivat, bevorzugt ein Carbomer enthält.

7. Steriles tropfbares mehrphasiges emulgatorfreies ophthalmisches Präparat nach einem der vorherigen Ansprüche, **dadurch gekennzeichnet, dass**
das Präparat ein ophthalmisch akzeptables, organisches Öl, vorzugsweise ein Triglycerid, bevorzugt ein mittelkettiges Triglycerid aufweist.

8. Steriles tropfbares mehrphasiges emulgatorfreies ophthalmisches Präparat nach einem der vorherigen Ansprüche, **dadurch gekennzeichnet, dass**
das Präparat wenigstens einen ophthalmischen Wirkstoff enthält, vorzugsweise eine Vitamin A-Komponente, bevorzugt Vitamin A-palmitat, besonders bevorzugt in Kombination mit einem Antioxidans wie Vitamin E.

9. Steriles tropfbares mehrphasiges emulgatorfreies ophthalmisches Präparat nach einem der vorherigen Ansprüche, **dadurch gekennzeichnet, dass**
das Präparat Sorbitol aufweist.

10. Steriles tropfbares mehrphasiges emulgatorfreies ophthalmisches Präparat nach einem der vorherigen Ansprüche, **dadurch gekennzeichnet, dass**
das Präparat Natriumhydroxid aufweist.

11. Steriles tropfbares mehrphasiges emulgatorfreies ophthalmisches Präparat nach einem der vorherigen Ansprüche, **dadurch gekennzeichnet, dass**
das Präparat Konservierungsmittel aufweist, vorzugsweise ausgewählt aus der Gruppe umfassend Cetrimid, Benzododeciniumchlorid, Benzalkoniumchlorid, Alexidin und/oder Thiomersal, bevorzugt ausgewählt aus der Gruppe umfassend Cetrimid und/oder Alexidin.

12. Steriles tropfbares mehrphasiges emulgatorfreies ophthalmisches Präparat nach einem der vorherigen Ansprüche; **dadurch gekennzeichnet, dass**
das Präparat Glycerol aufweist, vorzugsweise im Bereich von ≥ 0,01 Gew.-% bis ≤ 1,0 Gew.-%, bezogen auf das Gesamtgewicht des Präparats, bevorzugt im Bereich von ≥ 0,1 Gew.% bis ≤ 0,5 Gew.-%, vorzugsweise im Bereich von ≥ 0,2 Gew.% bis ≤ 0,3 Gew.-%.

13. Steriles tropfbare mehrphasiges emulgatorfreies ophthalmisches Präparat nach einem der vorherigen Ansprüche, **dadurch gekennzeichnet, dass**
das Präparat eine Osmolalität im Bereich im Bereich von ≥ 100 mosmol/kg bis ≤ 500 mosmol/kg, vorzugsweise im Bereich von ≥ 200 mosmol/kg bis ≤ 300 mosmol/kg, bevorzugt im Bereich von ≥ 220 mosmol/kg bits ≤ 260 mosmol/kg, aufweist.

14. Steriles tropfbares mehrphasiges emulgatorfreies ophthalmisches Präparat nach einem der vorherigen Ansprüche, **dadurch gekennzeichnet, dass**
das Präparat einen pH-Wert im Bereich von ≥ 6 bis ≤ 8, bevorzugt im Bereich von ≥ 6,8 bis ≤ 7,2, aufweist.

15. Steriles tropfbares mehrphasiges emulgatorfreies ophthalmisches Präparat nach einem der vorherigen Ansprüche, **dadurch gekennzeichnet, dass**
das Präparat Stoffe ausgewählt aus der Gruppe umfassend Polyacrylsäure, polymere Acrylsäurederivate, vorzugsweise Carbomere, Triglyceride, vorzugsweise mittelkettige Triglyceride, wasserlösliche Phosphatsalze, vorzugsweise Na₂HPO₄ x 12 H₂O, eine Vitamin A-Komponente, vorzugsweise Vitamin A-palmitat, Vitamin E, vorzugsweise D,L-α-Tocopherol, Sorbitol, Natriumhydroxid, Konservierungsmittel, vorzugsweise Cetrimid, Benzododeciniumchlorid, Benzalkoniumchlorid oder Alexidin, Glycerol und/oder Wasser aufweist.

16. Steriles tropfbares mehrphasiges emulgatorfreies ophthalmisches Präparat nach einem der vorherigen Ansprüche, **dadurch gekennzeichnet, dass**
das Präparat, bezogen auf das Gesamtgewicht des Präparats, die nachstehenden Stoffe umfasst:
a. 0,2 Gew.% polymere Acrylsäure;
b. 4,0 Gew.% Sorbitol;
c. 0,1 Gew.-% Na₂HPO₄ x 12 H₂O;
d. 0,0665 Gew.-% Natriumhydroxid;
e. 1,0 Gew.% mittelkettige Triglyceride;
f. ad 100 Gew.-% Wasser.

17. Verfahren zur Herstellung eines sterilen tropfbaren mehrphasigen emulgatorfreien ophthalmischen Präparats nach einem der Ansprüche 1 bis 16,
**dadurch gekennzeichnet, dass**
man eine flüssige hydrophobe Phase, insbesondere ein mittelkettiges Triglycerid, homogen in einer kontinuierlichen wässrigen Phase, insbesondere einem zuvor hergestellten sterilen Hydrogel, dispergiert.

18. Verfahren zur Herstellung eines Präparates nach Anspruch 17,
**dadurch gekennzeichnet, dass**
man eine Polyacrylsäure und/oder ein polymeres Acrylsäurederivat, bevorzugt ein Carbomer, einsetzt, die durch Neutralisation der Carboxylgruppen mit einer geeigneten Base, insbesondere Natriumhydroxidlösung, zur Gelbildung umgesetzt wird.

19. Verfahren zur Herstellung eines Präparates nach Anspruch 17 oder 18,
**dadurch gekennzeichnet, dass**
man der wässrigen Phase wenigstens ein wasserlösliches Phosphatsalz zusetzt.

20. Verfahren zur Herstellung eines Präparates nach einem der vorherigen Ansprüche,
**dadurch gekennzeichnet, dass**
man dem sterilen Präparat einen Wirkstoff, vorzugsweise Vitamin A, unter aseptischen Bedingungen zugesetzt und homogen einmischt.

21. Verwendung eines sterilen tropfbaren mehrphasigen emulgatorfreien ophthalmischen Präparats nach einem der vorherigen Ansprüche zur Herstellung eines kosmetischen und/oder pharmazeutischen Mittels zur Behandlung von Krankheiten oder Zuständen des Auges oder der das Auge umgebenden oder damit zusammenhängenden Organe oder Gewebe, insbesondere von trockenem Auge.

22. Mittel zur Behandlung von Krankheiten oder Zuständen des Auges oder der das Auge umgebenden oder damit zusammenhängenden Organe oder Gewebe, insbesondere in Form eines Gelpräparats, umfassend Stoffe gemäß einem der vorherigen Ansprüche.

23. Einmaldosis-Behältnis enthaltend ein steriles tropfbares mehrphasiges emulgatorfreies ophthalmisches Präparat nach einem der vorherigen Ansprüche.

24. Einmaldosis-Behältnis nach Anspruch 23, **dadurch gekennzeichnet, dass** das ophthalmische Präparat frei von Konservierungsmitteln ist.

25. Mehrdosen-Behältnis enthaltend ein steriles tropfbares mehrphasiges emulgatorfreies ophthalmisches Präparat nach einem der vorherigen Ansprüche.

26. Mehrdosen-Behältnis nach Anspruch 25, **dadurch gekennzeichnet, dass** das ophthalmische Präparat frei von Konservierungsmitteln ist.

## Claims

1. Sterile, droppable multiphase emulsifier-free ophthalmic preparation, in particular gel preparation, containing at least a liquid aqueous phase and at least a liquid hydrophobic phase,
**characterized in that**
the preparation contains at least one phosphate salt and has a viscosity in the range from ≥ 200 mPa·s to ≤ 2000 mPa·s that is determined according to the cone-plate method at 20 °C.

2. Sterile, droppable multiphase emulsifier-free ophthalmic preparation according to claim 1, **characterized in that**
the viscosity is in the range from ≥ 300 mPa·s to ≤ 1800 mPa·s, preferably in the range from ≥400 mPa·s to ≤ 1500 mPa·s, preferably in the range from ≥ 500 mPa·s to ≤ 1400 mPa·s, more preferably in the range from ≥ 600 mPa·s to ≤ 1350 mPa·s, particularly preferably in the range from ≥ 650 mPa·s to ≤ 1300 mPa·s.

3. Sterile, droppable multiphase emulsifier-free ophthalmic preparation according to claim 1 or 2, **characterized in that**
a water-soluble phosphate salt is selected from the group comprising alkali phosphate salts, alkali earth phosphate salts, ammonium phosphate salts, monohydrogen or dihydrogen phosphates thereof, and/or mixtures thereof, preferably selected from the group comprising sodium dihydrogen phosphate, sodium monohydrogen phosphate, potassium dihydrogen phosphate, potassium monohydrogen phosphate, and/or mixtures thereof.

4. Sterile, droppable multiphase emulsifier-free ophthalmic preparation according to any of the preceding claims, **characterized in that**
the preparation contains Na₂HPO₄ x 12 H₂O in the range from ≥ 0,01 weight-% to ≤ 5,0 weight-%, based on the total weight of the preparation, preferably in the range from ≥ 0,05 weight-% to ≤ 0,5 weight-%, more preferably in the range from ≥ 0,08 weight-% to ≤ 0,2 weight-%, particularly preferably about 0,1 weight-% Na₂HPO₄ x 12 H₂O.

5. Sterile, droppable multiphase emulsifier-free ophthalmic preparation according to any of the preceding claims, **characterized in that**
the preparation contains the aqueous phase as continuous phase and the hydrophobic phase as droplets dispersed therein.

6. Sterile, droppable multiphase emulsifier-free ophthalmic preparation according to any of the preceding claims, **characterized in that**
the preparation contains at least a polymeric gel-forming component, preferably a polyacrylic acid, and/or a polymeric acrylic acid derivative, preferably a carbomer.

7. Sterile, droppable multiphase emulsifier-free ophthalmic preparation according to any of the preceding claims, **characterized in that**
the preparation contains an ophthalmically acceptable organic oil, preferably triglyceride, more preferably a triglyceride of medium chain length.

8. Sterile, droppable multiphase emulsifier-free ophthalmic preparation according to any of the preceding claims, **characterized in that**
the preparation contains at least an ophthalmic active agent, preferably a Vitamin A component, more preferably Vitamin A palmitate, especially preferably in combination with an antioxidant, such as Vitamin E.

9. Sterile, droppable multiphase emulsifier-free ophthalmic preparation according to any of the preceding claims, **characterized in that**
the preparation comprises sorbitol.

10. Sterile, droppable multiphase emulsifier-free ophthalmic preparation according to any of the preceding claims, **characterized in that**
the preparation contains sodium hydroxide.

11. Sterile, droppable multiphase emulsifier-free ophthalmic preparation according to any of the preceding claims, **characterized in that**
the preparation comprises preservatives, preferably selected from the group comprising cetrimide, benzododecinium chloride, benzalkonium chloride, alexidine, and/or thiomersal, more preferably selected from the group comprising cetrimide and/or alexidine.

12. Sterile, droppable multiphase emulsifier-free ophthalmic preparation according to any of the preceding claims, **characterized in that**
the preparation comprises glycerol, preferably in the range from ≥ 0,01 weight-% to ≥ 1,0 weight-%, based on the total weight of the preparation, more preferably in the range from ≥ 0,1 weight-% to ≤ 0,5 weight-%, particularly preferably in the range from ≥ 0,2 weight-% to ≤ 0,3 weight-%.

13. Sterile, droppable multiphase emulsifier-free ophthalmic preparation according to any of the preceding claims, **characterized in that**
the preparation has an osmolality in the range from ≥100 mosmol/kg to ≤ 500 mosmol/kg, preferably in the range from ≥ 200 mosmol/kg to ≤ 300 mosmol/kg, more preferably in the range from ≥ 220 mosmol/kg to ≤ 260 mosmol/kg.

14. Sterile, droppable multiphase emulsifier-free ophthalmic preparation according to any of the preceding claims, **characterized in that**
the preparation has a pH value in the range from ≥ 6 to ≤ 8, preferably in the range from ≥ 6,8 to ≤7,2.

15. Sterile, droppable multiphase emulsifier-free ophthalmic preparation according to any of the preceding claims, **characterized in that**
the preparation contains compounds selected from the group comprising polyacrylic acid, polymeric acrylic acid derivatives, preferably carbomers, triglycerides, preferably triglycerides of medium chain length, water-soluble phosphate salts, preferably Na₂HPO₄ x 12 H₂O, a Vitamin A component, preferably Vitamin A palmitate, Vitamin E, preferably D,L-α-tocopherol, sorbitol, sodium hydroxide, preservatives, preferably cetrimide, benzododecinium chloride, benzalkonium chloride, or alexidine, glycerol, and/or water.

16. Sterile, droppable multiphase emulsifier-free ophthalmic preparation according to any of the preceding claims, **characterized in that**
the preparation comprises the following compounds, based on the total weight of the preparation:
a. 0,2 weight-% polymeric acrylic acid;
b. 4,2 weight-% sorbitol;
c. 0,1 weight-% Na₂HPO₄ x 12 H₂O;
d. 0,0665 weight-% sodium hydroxide;
e. 1,0 weight-% triglycerides of medium chain length;
f. ad 100 weight-% water.

17. Method for the preparation of a sterile, droppable multiphase emulsifier-free ophthalmic preparation according to any of claims 1-16,
**characterized in that**
a liquid hydrophobic phase, in particular a triglyceride of medium chain length is homogenously dispersed in a continuous aqueous phase, in particular a sterile hydrogel prepared previously.

18. Method for manufacturing a preparation according to claim 17,
**characterized in that**
a polyacrylic acid and/or a polymeric acrylic acid derivative, preferably a carbomer, is used that is reacted for gel-forming by neutralizing the carboxylic acid groups with an appropriate base, in particular a solution of sodium hydroxide.

19. Method for manufacturing a preparation according to claim 17 or 18,
**characterized in that**
at least a water-soluble phosphate salt is added to the aqueous phase.

20. Method for manufacturing a preparation according to any of the preceding claims,
**characterized in that**
an active agent, preferably Vitamin A, is added to the sterile preparation under aseptic conditions and is homogenously ad mixed.

21. Use of a sterile, droppable multiphase emulsifier-free ophthalmic preparation according to any of the preceding claims for the manufacture of a cosmetic and/or pharmaceutical agent for the treatment of diseases or conditions of the eye or organs or tissue surrounding the eye or associated with the eye, especially of dry eye syndrome.

22. Agent for the treatment of diseases or conditions of the eye or of organs or tissue surrounding the eye or associated with the eye, especially in the form of a gel preparation, comprising compounds according to any of the preceding claims.

23. Single-dosed container containing a sterile, droppable multiphase emulsifier-free ophthalmic preparation according to any of the preceding claims.

24. Single-dosed container, according to claim 23, **characterized in that** the ophthalmic preparation is free of preservatives.

25. Multi-dosed container containing a sterile, droppable multiphase emulsifier-free ophthalmic preparation according to any of the preceding claims.

26. Multi-dosed container, according to claim 25, **characterized in that** the ophthalmic preparation is free of preservatives.

## Revendications

1. Préparation ophtalmologique stérile, multiphase, exempte d'émulsifiant, coulant en gouttes, notamment préparation sous forme de gel, comportant au moins une phase liquide aqueuse et au moins une phase liquide hydrophobe, **caractérisée en ce que** ladite préparation contient au moins un sel de phosphate et présente une viscosité, déterminée selon la méthode cône et plateau à une température de 20 C, comprise entre ≥ 200 mPa·s et < 2000 mPa·s.

2. Préparation ophtalmologique stérile, multiphase, exempte d'émulsifiant, coulant en gouttes selon la revendication 1, **caractérisée en ce que** sa viscosité est comprise entre ≥ 300 mPa·s et ≤ 1800 mPa·s, de préférence comprise entre ≥ 400 mPa·s et ≤ 1500 mPa·s, de préférence encore comprise entre ≥ 500 mPa·s et ≤ 1400 mPa·s, de manière particulièrement préférée comprise entre ≥ 600 mPa·s et ≤ 1350 mPa·s, de manière tout à fait préférée comprise entre ≥ 650 mPa·s et ≤ 1300 mPa·s.

3. Préparation ophtalmologique stérile, multiphase, exempte d'émulsifiant, coulant en gouttes selon la revendication 1 ou la revendication 2, **caractérisée en ce qu'**un sel de phosphate soluble dans l'eau est choisi dans le groupe comportant les sels de phosphate alcalins, les sels de phosphate alcalinoterreux, les sels de phosphate d'ammonium, les mono-ou dihydrogénophosphates de ceux-ci et/ou les mélanges de ceux-ci, de préférence choisi dans le groupe comprenant le dihydrogénophosphate de sodium, le monohydrogénophosphate de sodium, le dihydrogénophosphate de potassium, le monohydrogénophosphate de potassium et/ou les mélanges de ceux-ci.

4. Préparation ophtalmologique stérile, multiphase, exempte d'émulsifiant, coulant en gouttes selon l'une quelconque des revendications précédentes, **caractérisée en ce que** ladite préparation contient entre ≥ 0,01 % en poids et ≤ 5,0 % en poids de Na₂HPO₄ x 12 H₂O rapporté au poids total de la préparation, de préférence entre ≥0,05 % en poids et ≤ 0,5 % en poids, de préférence encore entre ≥ 0,08 % en poids et ≤ 0,2 % en poids, et de manière particulièrement préférée environ 0,1 % en poids de Na₂HPO₄ x 12 H₂O.

5. Préparation ophtalmologique stérile, multiphase, exempte d'émulsifiant, coulant en gouttes selon l'une quelconque des revendications précédentes, **caractérisée en ce que** la phase aqueuse de la préparation est une phase continue et la phase hydrophobe consiste en des gouttelettes dispersées dans ladite phase aqueuse.

6. Préparation ophtalmologique stérile, multiphase, exempte d'émulsifiant, coulant en gouttes selon l'une quelconque des revendications précédentes, **caractérisée en ce que** ladite préparation contient au moins un composant gélifiant polymère, de préférence un acide polyacrylique et/ou un dérivé d'acide acrylique polymère, de préférence un carbomère.

7. Préparation ophtalmologique stérile, multiphase, exempte d'émulsifiant, coulant en gouttes selon l'une quelconque des revendications précédentes, **caractérisée en ce que** ladite préparation contient une huile organique ophtalmologiquement acceptable, de préférence un triglycéride, de préférence encore un triglycéride à chaîne moyenne.

8. Préparation ophtalmologique stérile, multiphase, exempte d'émulsifiant, coulant en gouttes selon l'une quelconque des revendications précédentes, **caractérisée en ce que** ladite préparation contient au moins un principe actif ophtalmologique, de préférence un composant de la vitamine A, de préférence le palmitate de vitamine A, de manière particulièrement préférée associé à un antioxydant tel que la vitamine E.

9. Préparation ophtalmologique stérile, multiphase, exempte d'émulsifiant, coulant en gouttes selon l'une quelconque des revendications précédentes, **caractérisée en ce que** ladite préparation contient du sorbitol.

10. Préparation ophtalmologique stérile, multiphase, exempte d'émulsifiant, coulant en gouttes selon l'une quelconque des revendications précédentes, **caractérisée en ce que** ladite préparation contient de l'hydroxyde de sodium.

11. Préparation ophtalmologique stérile, multiphase, exempte d'émulsifiant, coulant en gouttes selon l'une quelconque des revendications précédentes, **caractérisée en ce que** ladite préparation contient des conservateurs, de préférence choisis dans le groupe comportant le cétrimide, le chlorure de benzododécinium, le chlorure de benzalkonium, l'alexidine et/ou le thiomersal, de préférence encore choisis dans le groupe comportant le cétrimide et/ou l'alexidine.

12. Préparation ophtalmologique stérile, multiphase, exempte d'émulsifiant, coulant en gouttes selon l'une quelconque des revendications précédentes, **caractérisée en ce que** ladite préparation contient du glycérol, de préférence entre ≥ 0,01 % en poids et ≤ 1,0 % en poids rapporté au poids total de la préparation, de préférence encore entre ≥ 0,1 % en poids et ≤ 0,5 % en poids, et de préférence entre ≥ 0,2 % en poids et ≤ 0,3 % en poids.

13. Préparation ophtalmologique stérile, multiphase, exempte d'émulsifiant, coulant en gouttes selon l'une quelconque des revendications précédentes, **caractérisée en ce que** ladite préparation présente une osmolalité comprise entre ≥ 100 mOsmol/kg et ≤ 500 mOsmol/kg, de préférence entre ≥ 200 mOsmol/kg et ≤ 300 mOsmol/kg, de préférence encore entre ≥220 mOsmol/kg et ≤ 260 mOsmol/kg.

14. Préparation ophtalmologique stérile, multiphase, exempte d'émulsifiant, coulant en gouttes selon l'une quelconque des revendications précédentes, **caractérisée en ce que** ladite préparation a un pH compris entre ≥ 6 et ≤ 8, de préférence compris entre ≥ 6,8 et ≤ 7,2.

15. Préparation ophtalmologique stérile, multiphase, exempte d'émulsifiant, coulant en gouttes selon l'une quelconque des revendications précédentes, **caractérisée en ce que** ladite préparation contient des substances choisies dans le groupe comportant l'acide polyacrylique, les dérivés d'acide acrylique polymère, de préférence les carbomères, les triglycérides, de préférence les triglycérides à chaîne moyenne, les sels de phosphate solubles dans l'eau, de préférence le Na₂HPO₄ x 12 H₂O, un composant de la vitamine A, de préférence le palmitate de vitamine A, la vitamine E, de préférence le D,L-α-tocophérol, le sorbitol, l'hydroxyde de sodium, les conservateurs, de préférence le cétrimide, le chlorure de benzododécinium, le chlorure de benzalkonium ou l'alexidine, le glycérol et/ou l'eau.

16. Préparation ophtalmologique stérile, multiphase, exempte d'émulsifiant, coulant en gouttes selon l'une quelconque des revendications précédentes, **caractérisée en ce que** ladite préparation comprend les substances suivantes :
a. 0,2 % en poids d'acide acrylique polymère ;
b. 4,0 % en poids de sorbitol ;
c. 0,1 % en poids de Na₂HPO₄ x 12 H₂O ;
d. 0,0665 % en poids d'hydroxyde de sodium ;
e. 1,0 % en poids de triglycérides à chaîne moyenne ;
f. eau, q.s. ad 100 % en poids;
les pourcentages étant rapportés au poids total de la préparation.

17. Procédé de fabrication d'une préparation ophtalmologique stérile, multiphase, exempte d'émulsifiant, coulant en gouttes selon l'une quelconque des revendications 1 à 16, **caractérisé en ce que** l'on réalise la dispersion homogène d'une phase liquide hydrophobe, notamment un triglycéride à chaîne moyenne, dans une phase aqueuse continue, notamment dans un hydrogel stérile fabriqué au préalable.

18. Procédé de fabrication d'une préparation selon la revendication 17, **caractérisé en ce que** l'on utilise un acide polyacrylique et/ou un dérivé d'acide acrylique polymère, de préférence un carbomère, que l'on fait réagir, en vue de la gélification, par neutralisation des groupes carboxyliques avec une base adaptée, notamment une solution d'hydroxyde de sodium.

19. Procédé de fabrication d'une préparation selon la revendication 17 ou la revendication 18, **caractérisé en ce que** l'on ajoute à la phase aqueuse au moins un sel de phosphate soluble dans l'eau.

20. Procédé de fabrication d'une préparation selon l'une quelconque des revendications précédentes, **caractérisé en ce que** l'on mélange à la préparation stérile, en conditions d'asepsie et de manière homogène, un principe actif, de préférence de la vitamine A.

21. Utilisation d'une préparation ophtalmologique stérile, multiphase, exempte d'émulsifiant, coulant en gouttes selon l'une quelconque des revendications précédentes, pour la fabrication d'un agent cosmétique et/ou pharmaceutique destiné à traiter des maladies ou affectations de l'oeil ou des organes ou tissus entourant l'oeil ou associés à lui, notamment l'oeil sec.

22. Agent destiné à traiter des maladies ou affections de l'oeil ou des organes ou tissus entourant l'oeil ou associés à lui, notamment sous la forme d'une préparation en gel, comprenant des substances selon l'une quelconque des revendications précédentes.

23. Récipient monodose contenant une préparation ophtalmologique stérile, multiphase, exempte d'émulsifiant, coulant en gouttes selon l'une quelconque des revendications précédentes.

24. Récipient monodose selon la revendication 23, **caractérisé en ce que** la préparation ophtalmologique est exempte de conservateurs.

25. Récipient multidose contenant une préparation ophtalmologique stérile, multiphase, exempte d'émulsifiant, coulant en gouttes selon l'une quelconque des revendications précédentes.

26. Récipient multidose selon la revendication 25, **caractérisé en ce que** la préparation ophtalmologique est exempte de conservateurs.
